# EUROPEAN PATENT APPLICATION

(11) **EP 4 653 002 A2**
(43) Date of publication of application: **26.11.2025**
(21) Application number: 25203479.8
(22) Date of filing: 23.03.2021
(51) Int. Cl.: A61K 31/724

(54) **ORAL TERPENE CYCLODEXTRIN INCLUSION COMPLEX VEHICLES**

(30) Priority: 23.03.2020 US 202062993346 P
(62) Divisional of application: 21774359.0
(71) Applicant: Czap Research And Development, LLC, Napa, California 94558 (US)
(72) Inventor: CZAP, Al, Napa, 94558 (US)
(74) Representative: De Clercq & Partners

(57) **Abstract**

The invention provides oral terpene cyclodextrin inclusion complex delivery vehicles, including formulations in which the cyclodextrin inclusion complex is provided together with enzyme having a cyclodextrin-degrading activity capable of digesting the cyclodextrin, so that upon delivery of the vehicle to a target the enzyme is activated and releases the guest molecule from the cyclodextrin cavity. In alternative aspects, these cyclodextrin inclusion complex delivery vehicles are for example provided in the form of time release formulations, for example for treatment of airway mucus dysfunction. Formulations are also provided with erectogenic efficacy.

## Description

### FIELD OF THE INVENTION

The invention is in the field of biochemical constructs for delivery of bioactive terpenes, including camphene, as inclusions within cyclodextrins in oral formulations that may include enzymes having cyclodextrin-degrading activities.

### BACKGROUND OF THE INVENTION

Cyclodextrins are non-reducing cyclic glucose oligosaccharides, frequently the product of cyclomaltodextrin glucanotransferase (E.C. 2.4.1.19; CGTase) catalyzed degradation of starch. Cyclodextrins may have a variety of structures (see Saenger et al., Chem. Rev. 98 (1998) 1787-1802), including three common cyclodextrins with 6, 7 or 8 D-glucopyranonsyl residues (α-, β-, and γ-cyclodextrin respectively) linked in a ring by α-1,4 glycosidic bonds. The frustoconical shape of cyclodextrins forms a cavity or lumen, with the cavities having different diameters depending on the number of glucose units. The scale of selected cyclodextrin (CD) structures is set out in Table 1. Larger cyclodextrins such as cyclomaltononaose (δ-CD) and cyclomaltodecaose (ε-CD) are also possible, as well as a variety of cyclodextrin-based supra-molecular structures (see Zhang and Ma, Adv Drug Deliv Rev. 2013 Aug;65(9):1215-33).

**Table 1: cyclodextrin structures**

| Cyclodextrin | Lumen diameter (nm) | |
|---|---|---|
| | Inner rim | Outer rim |
| α, (glucose)₆ | 0.45 | 0.53 |
| β, (glucose)₇ | 0.60 | 0.65 |
| γ, (glucose)₈ | 0.75 | 0.85 |

Cyclodextrins are generally amphipathic, with the wider rim of the lumen displaying the 2- and 3-OH groups and the narrower rim displaying 6-OH. These hydrophilic hydroxyl groups are accordingly on the outside of the lumen, whereas the inner surface is generally hydrophobic and lined with the anomeric oxygen atoms and the C3-H and C5-H hydrogen atoms. In aqueous solution, this hydrophobic lumen may contain water molecules, for example about 3 (α-CD), 7 (β-CD) or 9 (γ-CD) poorly held but low entropy, and hence relatively easily displaceable water molecules. Thus, otherwise hydrophilic cyclodextrins may bind retain one or more suitably-sized molecules within, or partially within, the lumen of the CD, forming a cyclodextrin inclusion body or complex. For example, non-polar aliphatic and aromatic compounds, including drugs, such as lipophilic drugs, may be bound so as to increase the water solubility of normally hydrophobic compounds or minimize undesirable properties such as odor or taste in certain food additives. For this reason, cyclodextrin inclusions are widely used in the pharmaceutical, food and cosmetic fields (see Hedges, Chem. Rev. 98 (1998) 2035-2044). Cyclodextrins have for example been used in a variety of sustained release drug preparations, such as for inclusion complexes of a medical compound with a hydrophobic cyclodextrin derivative (U.S. Patent No. 4,869,904).

Cyclodextrins may be chemically modified in a wide variety of ways. For example, to modify the inclusion specificity, physical and chemical properties of the cyclodextrin. Hydroxyl groups of a CD may for example be derivatized. For example, two modified CDs have been used in a number of pharmaceutical products: SBE-β-CD, or Captisol, a polyanionic variably substituted sulfobutyl ether of β-CD, and HP-β-CD, a modified CD commercially developed by Janssen. Additional CD derivatives include sugammadex or Org-25969, in which the 6-hydroxy groups on γ-CD have been replaced by carboxythio acetate ether linkages, and hydroxybutenyl-β-CD. Alternative forms of cyclodextrin include: 2,6-Di-O-methyl-β-CD (DIMEB), 2-hydroxylpropyl-β-cyclodextrin (HP-β-CD), randomly methylated-β-cyclodextrin (RAMEB), sulfobutyl ether β-cyclodextrin (SBE-β-CD), and sulfobutylether-γ-cyclodextrin (SBEγCD), sulfobutylated beta-cyclodextrin sodium salt, sulfobutylated beta-cyclodextrin sodium salt, (2-Hydroxypropyl)-alpha-cyclodextrin, (2-Hydroxypropyl)-beta-cyclodextrin, (2-Hydroxypropyl)-gamma-cyclodextrin, DIMEB-50 Heptakis(2,6-di-O-methyl)-beta-cyclodextrin, TRIMEB Heptakis(2,3,6-tri-O-methyl)-beta-cyclodextrin, methyl-beta-cyclodextrin, octakis(6-deoxy-6-iodo)-gamma-cyclodexrin, and, octakis(6-deoxy-6-bromo)-gamma-cyclodexrin. Although CDs such as these have been developed with favorable pharmacological and toxicological profiles, there is the potential that, following administration, residual CDs may perturb the pharmacokinetic properties of drugs, including coadministered drugs, particularly after parenteral administration (see Stella and He, Toxicol Pathol January 2008 vol. 36 no. 1 30-42).

Cyclodextrins are variably susceptible to enzymatic digestion. For example, γ-CD is relatively easily hydrolyzed by α-amylases whereas α-cyclodextrin is more poorly hydrolyzed. CD based therapeutics generally depend on the activity of endogenous amylases to digest the CD. There is however significant variability in amylase activity between patients. For example, patients with pancreatic insufficiency, cystic fibrosis, celiac disease or Crohn's disease, may lack normal amounts of amylase. Similarly, patients, particularly geriatric patients, may be deficient in gastric acid production and thereby fail to create conditions of appropriately low pH in the duodenum to properly trigger release of pancreatic amylase. A similar effect may result from the increasing common use of antacids, histamine-2 blockers, proton pump inhibitors or alternative acid blockers.

A variety of microbial cyclodextrin digesting enzymes have been identified. CD-degrading enzymes include cyclomaltodextrinase (or cyclodextrinase, or CDase, EC 3.2.1.54), maltogenic amylase (EC 3.2.1.133), neopullulanase (EC 3.2.1.135), which have been reported to be capable of hydrolyzing CDs and in some cases additional substrates such as pullulan, and starch. Cyclodextrinase (CDase) catalyzes the hydrolysis of CDs to form linear oligosaccharides of α-1,4-linkages, and it can accordingly release substances from CD inclusion complexes. A CDase from *Bacillus macerans* was reported in 1968, and many CDases from bacteria have since been characterized, such as enzymes from *Bacillus sp., Thermoanaerobacter ethanolicus* strain 39E, *Flavobacterium sp.* , and *Klebsiella oxytoca* strain M5a1. Archaea CDases have been characterized from *Archaeoglobus fulgidus, Thermococcus sp.* B1001, *Thermococcus sp.* CL1, *Thermofilum pendens,* and *Pyrococcus furiosus.* The structure of the CDase from *Flavobacterium sp.* has been characterized in detail (see Sun et al., Archaea, Volume 2015 (2015), Article ID 397924, reporting the identification of a gene encoding a cyclodextrinase from *Thermococcus kodakarensis* KOD1 (CDase-Tk)).

Camphene is a monoterpene with a bicyclic skeleton that is bicyclo[2.2.1]heptane substituted by geminal methyl groups at position 2 and a methylidene group at position 3. It is a widespread natural product found in many essential oils, typically as a racemic mixture in various proportions of D-camphene and L-camphene (Ochocka et al., 2002, Pharmaceutical Biology, 40:5, 395-399). It has been widely used as a flavouring and a fragrance. Camphene has also been described as having various physiological effects, such as hypolipidemic effects (Vaillianou et al., PLoS One, 2011; 6(11)e20516).

Airway mucus dysfunction contributes to, or is symptomatic of, a wide variety of airway diseases and conditions (see Fahy & Dickey, 2010), often manifesting as cough or dyspnea. Diseases characterized by airway mucus dysfunction include cystic fibrosis, asthma, chronic obstructive pulmonary disease, primary ciliary dyskinesia, non-cystic fibrosis bronchiectasis, panbronchiolitis, various immunodeficiency states. Conditions associated with airway mucus dysfunction may include various disruptions of normal lung mechanics, for example in intubated, paralyzed, immobilized or post-surgical patients, where problems may be associated with retained mucus.

### SUMMARY OF THE INVENTION

Camphene-containing cyclodextrin inclusion complex delivery vehicles are provided, for example for use in treating airway mucus dysfunction, or for providing an erectogenic effect in a male subject. In addition to camphene, formulations may include additional inclusion complex guest molecules, including additional terpenes, such as botanical terpenes. The additional guest molecules may for example include: eucalyptol (i.e. 1,3,3-Trimethyl-2-oxabicyclo[2.2.2]octane; CAS 470-82-06; 1,8-cineole; 1,8-epoxy-p-menthane); guaiol (i.e. 2-[(3S,5R,8S)-3,8-Dimethyl-1,2,3,4,5,6,7,8-octahydro-5-azulenyl]-2-propanol; CAS 489-86-1; champacol), and/or delta-3 carene (i.e. 3,7,7-trimethylbicyclo[4.1.0]hept-3-ene; CAS 13466-78-9). Additional guest molecules may for example be provided in CD inclusions of botanical extracts or compositions, such as peppermint and/or fenugreek compositions, such as peppermint oils or fenugreek powders.

A biologically acceptable carrier may be provided for the cyclodextrin inclusion complex, so that the guest molecule is stably retained by the cyclodextrin within the biologically acceptable carrier. An enzyme may also be provided in the vehicle, having a cyclodextrin-degrading activity capable of digesting the cyclodextrin retaining the guest molecule. The enzyme may be formulated so that the cyclodextrin-degrading activity is activated on delivery of the vehicle to a target so as to release the guest molecule from the cyclodextrin cavity.

In alternative aspects of the delivery vehicle, the enzyme may be co-formulated with the cyclodextrin inclusion complex or the enzyme may be co-packaged in the delivery vehicle with the cyclodextrin inclusion complex. When the enzyme is co-packaged, the delivery vehicle may further include a biochemically acceptable carrier for the enzyme.

The enzyme may for example be an amylase, a cyclodextrinase, maltogenic amylase or neopullulanase. An amylase may for example be a mammalian salivary amylase or a pancreatic amylase, or an amylase of fungal, or bacterial origin. A cyclodextrinase may for example be a microbial cyclodextrinase.

The cyclodextrin may for example be a CD derivative, such as a hydrophobic alkylated cyclodextrin or a mixed methylated/ethylated cyclodextrin.

The ratio of the cyclodextrin to the guest molecule may for example be 5:1, 4:1, 3:1, 2:1, 1:1, 1:2, 1:3, 1:4 or 1:5, although a wide range of alternative values for this parameter are also possible, including non-integer ratios.

The cyclodextrin may for example be an alpha, beta or gamma cyclodextrin, although again a very wide range of alternative CD structures may be used.

In select embodiments, additional guest molecules may for example be provided, such as a drug or pro-drug, a PDE5 inhibitor, a flavonoid (quercetin), cannabinoid or anti-inflammatory (including acetaminophen). In that circumstance the biologically acceptable carrier may advantageously be a pharmaceutically acceptable carrier. The delivery vehicle may be formulated for sustained release of the guest molecules.

In this way, the invention provides alternative embodiments in which CD delivery vehicles may be used as a medicament.

Methods are provided for treating patients having airway mucus dysfunctions that are symptomatic of a disease, such as cystic fibrosis, asthma, chronic obstructive pulmonary disease, primary ciliary dyskinesia, non-cystic fibrosis bronchiectasis, panbronchiolitis, or an immunodeficiency states. The airway mucus dysfunction may alternatively be symptomatic of a condition associated with a disruption of lung mechanics, such as intubation, paralysis, immobilization or surgical trauma.

Methods are also provided for providing an erectogenic effect in a male subject, for example a male over 50, 60 or 70 years old, or a male subject suffering from an erectile dysfunction.

### DETAILED DESCRIPTION OF THE INVENTION

Specific formulations of delivery vehicles may for example include cyclodextrin inclusion complex formulations made by combining cyclodextrin inclusions prepared from a number of sources, including natural botanical extract sources. For example formulations that combine, for example in a single formulation or in distinct formulations provided in combination, CD inclusion complexes prepared from 2, 3, 4, 5 or 6 of the following:
extract of eucalyptus *(Myrtaceae)* (for example standardized for eucalyptol 6 mg, or 4-8 mg), in a CD inclusion, for example in a beta CD or gamma CD inclusion; and/or,
extract of *Syncarpia glomulifera* (for example standardized for camphene 6 mg, or 4-8 mg), in a CD inclusion, for example in a beta CD or gamma CD inclusion; and/or,
peppermint *(Mentha piperita)* oil 6 mg, or 4-8 mg), in a CD inclusion, for example a beta CD or gamma CD inclusion; and/or,
extract of *Pinus* (for example *Pinaster* and/or *Elliotii* and/or *Sylvestris*), (for example standardized for Carene Delta 3 4mg, or 2-6 mg), in a CD inclusion, for example a beta CD or gamma CD inclusion; and/or,
fenugreek extract, for example fenugreek absolute (*Trigonella foenum-graecum*) for 4 mg, or 2-6 mg), in a CD inclusion, for example beta CD or gamma CD inclusion; and/or,
extract of *Bulnesia sarmientoi* (for example standardized for guaiol for 4 mg, or 2-6 mg), in a CD inclusion, for example beta CD or gamma CD inclusion.

Various ratios of ingredients may be provided, for example in 5-250 mg per dose of any one inclusion complex. Select formulations may be of exclusively botanically sourced active ingredients. Specific dosage forms may for example be as a single 0 capsule, sustained release, as follows:
extract of eucalyptus *(Myrtaceae)* (for example standardized for eucalyptol 6 mg, or 4-8 mg), in a CD inclusion, for example in a beta CD or gamma CD inclusion;
extract of *Syncarpia glomulifera* (for example standardized for camphene 6 mg, or 4-8 mg), in a CD inclusion, for example in a beta CD or gamma CD inclusion;
peppermint *(Mentha piperita)* oil 6 mg, or 4-8 mg), in a CD inclusion, for example a beta CD or gamma CD inclusion;
extract of *Pinus* [for example *Pinaster* and/or *Elliotii* and/or *Sylvestris*] (for example standardized for Carene Delta 3 4mg, or 2-6 mg), in a CD inclusion, for example a beta CD or gamma CD inclusion;
fenugreek extract, for example fenugreek absolute (*Trigonella foenum-graecum*) for 4 mg, or 2-6 mg), in a CD inclusion, for example beta CD or gamma CD inclusion;
extract of *Bulnesia sarmientoi* (for example standardized for guaiol for 4 mg, or 2-6 mg), in a CD inclusion, for example beta CD or gamma CD inclusion;
K250 time release agent 80 mg;
Amylase 7 mg;
Ca laurate 5 mg.

As used in the formulations herein, peppermint oil may be an extract of the peppermint plant *Mentha piperita.* The composition of peppermint oils varies, but has been characterized as comprising menthol (40.7%) and menthone (23.4%), (+/-)-menthyl acetate, 1,8-cineole, limonene, beta-pinene and beta-caryophyllene (Schmidt et al., 2009, Nat Prod Commun 4(8):1107; CAS Number 8006-90-4, MDL number MFCD00147870).

Fenugreek (Trigonella foenum-graecum) compositions are widely known, and are understood to comprise a variety of alkaloids, amino acids, saponins, steroidal sapinogens and flavonoids (Wani and Kumar, 2018, J. of the Saudi society of Agricultural Sciences 17(2): 97). For example, a fenugreek extract may be produced by solvent extracting the seeds of Trigonella foenum-graecum, sometimes identified as Fenugreek Absolute (CAS Number 84625-40-1, FEMA Number 2486, EC Number 283-415-1, MDL number MFCD01772302).

In select embodiments, guest molecules may be provided in the form of plant extracts, for example: eucalyptol from Eucalyptus (e.g Eucalyptus oil, CAS Number 8000-48-4, FEMA Number 2466, EC Number 283-406-2); camphene from Syncarpia; delta-3-carene (δ-3-carene) from pine tree (e.g. species: Pinus Pinaster; Pinus Elliotii; Pinus Sylvestris, cedar, basil, pine, rosemary, and bell pepper); guaiol from Bulnesia sarmientoi (a sesquiterpenoid alcohol, also found in cypress pine and guaiacum); peppermint oil containing menthol; fenugreek extract from Trigonella foenum graecum seeds. Alternative embodiments may for example comprise extracts, terpenes or oils from: Eucalyptus (Myrtaceae), Syncarpia glomulifera, Pinus (Pinaster and/or Elliotii and/or Sylvestris) and Bulnesia sarmientoi, Peppermint (Mentha piperita) and Fenugreek (Trigonella foenum-graecum). These extracts may for example be formulated in a beta and gamma dextrin fiber matrix, with high and low viscosity hydroxypropyl methylcellulose (sustained release), hypromellose (derived from cellulose) capsule, cellulose, calcium laurate and amylase (such as plant or microbial amylase).

In a select embodiment, formulations may for example comprise, for example in a plant cellulose capsule:
Eucalyptol (10% by wt) in beta CD inclusion 75 mg;
Camphene (10% by wt) in beta CD inclusion 75 mg;
Carene delta 3 (10% by wt) in beta CD inclusion 50 mg;
Guaiol (10% by wt) in gamma CD inclusion 50 mg;
Peppermint oil (15% by wt) in beta CD inclusion 50 mg;
Fenugreek Absolute (oil) (10% by wt) in gamma CD inclusion 50 mg;
K250 time release agent 80 mg;
Amylase 5 mg; and
Ca Laurate 5 mg.

A wide variety of biologically active compounds may be included in delivery vehicles of the invention, as additional distinct ingredients and/or as additional guest molecules, for example in the form of pharmaceutical compositions, such as: Docetaxel (US Patent Publications 20140336149, 20130296268); carbamazepine (US Patent Publication 20140080812); Rifampicin (US Patent No 7001893); cardiac glycosides, particularly digoxin (US Patent No 4555504); progesterone (see Zoppetti et al., Journal of Inclusion Phenomena and Macrocyclic Chemistry, April 2007, Volume 57, Issue 1, pp 283-288); albendazole; mebendazole; ricobendazole; fenoprofen; ketoprofen; cocaine; gliclazide; digitoxin; macrocyclic compounds (MCCs); ibuproxam; prochloro-methazine; DY- 9760e; NSC-639829; ETH-615; piroxicam; levemopamil HCl; ziprasdone mesylate; sulindac; mebendazole; sulindac; phenolphthalein; danazol (see Challa et al., 2005, AAPS PharmSciTech 2005; 6 (2) Article 43); itraconazole; nelfinavirmesylate; telmisartan; 5-fluorouracile and other nucleoside analogues; camptothecin; humulene (also known as α-humulene or α-caryophyllene); or, flavonoids.

A select embodiment is a medicinal food composition that comprises eucalyptol (e.g. ~10% by wt) in beta CD inclusion 75 mg; camphene (e.g. ~10% by wt) in beta CD inclusion 75 mg; carene delta 3 (e.g. ~10% by wt) in beta CD inclusion 50 mg; guaiol (e.g. ~10% by wt) in gamma CD inclusion 50 mg; peppermint oil (e.g. ~15% by wt) in beta CD inclusion 50 mg; fenugreek absolute (oil) (e.g. ~10% by wt) in gamma CD inclusion 50 mg; and humulene.

In select embodiments, the enzyme provided in the vehicle may be formulated so that the cyclodextrin-degrading activity is activated on delivery of the vehicle to a target so as to release the guest molecule from the cyclodextrin cavity. Enzyme activation may for example be accomplished in a medicament, for example for oral delivery, in a dry dosage form, such as a capsule or tablet, in which the enzyme is admixed, so that the enzyme will not be active until activated by moisture in the gastrointestinal tract of a host. Similarly, a wide variety of time release matrices and formulations are known, which may be adapted for use in CD delivery vehicles so as to orchestrate the appropriate activation of the CD-degrading enzyme upon delivery to the target.

In various aspects, CD delivery vehicles may have the enzyme co-formulated with the cyclodextrin inclusion complex, as for example discussed above, or the enzyme may be co-packaged in the delivery vehicle with the cyclodextrin inclusion complex. In the case of co-packaging, the delivery vehicle may for example include a biochemically acceptable carrier for the enzyme - distinct from the carrier for the CD inclusion complex. For example, delivery vehicles may be provided with separated compartments containing the CD inclusion complex and the CD-degrading enzyme, so that the delivery vehicle will be made up of a CD inclusion complex compartment connected to a CD-degrading enzyme compartment. Mechanisms may be provided for the combined release of the CD inclusion complex and the CD-degrading enzyme from the respective compartments in the delivery vehicle. For example, syringes may be provided having distinct compartments of this kind that are discharged by a common discharge mechanism, such as a mechanism that cooperatively displaces pistons in each compartment so as to discharge aliquots of CD inclusion complex and CD-degrading enzyme, so that the enzyme and the complex may then be comingled to activate the enzymatic release of the guest molecule from the CD. Vehicles of this kind may for example be used to dispense a topical cream or other surface-active formulations. A wide variety of delivery vehicles of this kind may be adapted from devices that are known for dispensing two-part compositions such as epoxy resins, two-part medicaments or dental formulations, as for example disclosed in U.S. Patent Nos. 4538920, 8100295, 8308340, 8875947, 8499976 and International Patent Publications WO2007041266 and WO2000021842.

There are a wide variety of techniques available to prepare CD inclusion complexes, as for example described in: Chaudhary & Patel, IJPSR, 2013, Vol. 4(1): 68-76; Carneiro et al., 2019, Int. J. Mol. Sci. 2019, 20, 642; US Patent Publications US20090029020; US20090214446; US Patent Nos 5,070,081; 5,552,378; 5,674,854, and 8,658,692. A common approach is known as the kneading method, which involves mixing CDs with water or an aqueous alcohol to provide a paste. The bioactive molecule may then be added to the paste and kneaded for a specified time. The kneaded mixture may then be dried and passed through sieve if desired. Alternatively, a slurry method involves steps of: mixing the bioactive molecule and the cyclodextrin, adding a suitable amount of water to the mixture, typically with vigorous mixing, until a paste or a slurry is formed; continuing the mixing with further addition of water if necessary to maintain the paste or the slurry consistency, for a suitable period of time, such as 15 minutes, to form the inclusion complex; and, drying the product of this final step. Other ingredients, such as emulsifiers, may facilitate the formation of inclusion complexes, for example processes that involve steps of: dry blending a cyclodextrin and an emulsifier (e.g., pectin); combining the dry blend of cyclodextrin and the emulsifier with a solvent such as water in a reactor, and agitating; adding the guest molecule and stirring (e.g., for approximately 5 to 8 hours); optionally cooling the reaction mixture with stirring; and emulsifying the mixture prior to drying the cyclodextrin inclusion complex to form a powder. Other known approaches to preparing CD inclusions involve lyophilization, microwave irradiation, and a supercritical fluid antisolvent technique.

The CD delivery vehicles of the invention can be provided alone or in combination with other compounds (for example, nucleic acid molecules, small molecules, peptides, or peptide analogues), in the presence of a carrier, such as a liposome, an adjuvant, or any pharmaceutically or biologically acceptable carrier. Select embodiments include medicaments in a form suitable for administration to animal hosts, such as mammals, for example, humans. As used herein "pharmaceutically acceptable carrier" or "excipient" includes any and all solvents, dispersion media, coatings, antibacterial and antifungal agents, isotonic and absorption delaying agents, and the like that are physiologically compatible. The carrier can be suitable for any appropriate form of administration, including topical, subcutaneous, intradermal, intravenous, parenteral, intraperitoneal, intramuscular, sublingual, inhalational, intratumoral or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the biologically active compound, use thereof in the pharmaceutical compositions of the invention is contemplated. Supplementary active compounds can also be incorporated into the compositions.

Conventional pharmaceutical practice may be employed to provide suitable formulations or compositions to administer the delivery vehicles to subjects. Any appropriate route of administration may be employed, for example, parenteral, intravenous, intradermal, subcutaneous, intramuscular, intracranial, intraorbital, ophthalmic, intraventricular, intracapsular, intraspinal, intrathecal, intracisternal, intraperitoneal, intranasal, inhalational, aerosol, topical, intratumoral, sublingual or oral administration. Therapeutic formulations may be in the form of liquid solutions or suspensions; for oral administration, formulations may be in the form of tablets or capsules; for intranasal formulations, in the form of powders, nasal drops, or aerosols; and for sublingual formulations, in the form of drops, aerosols or tablets.

Cyclodextrin-degrading or digesting enzymes may for example be formulated for oral delivery. Enteric enzyme formulations may for example be provided, such as submicron particle formulations prepared by emulsion solvent evaporation (Sharma et al., Pharm Dev Technol. 2013 May-Jun;18(3):560-9). Similarly, delivery vehicles may be formulated as hydrogels (see US Patent Publication 20140094433), or medicated gums (see US Patent Publication 20130022652).

Methods well known in the art for making formulations are found in, for example, "Remington's Pharmaceutical Sciences" (20th edition), ed. A. Gennaro, 2000, Mack Publishing Company, Easton, PA. Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

Pharmaceutical compositions of the present invention may be in any form which allows for the composition to be administered to a patient. For example, the composition may be in the form of a solid, liquid or gas (aerosol). Typical routes of administration include, without limitation, oral, topical, parenteral, sublingual, rectal, vaginal, and intranasal. The term parenteral as used herein includes subcutaneous injections, intravenous, intramuscular, epidural, intrasternal injection or infusion techniques. Pharmaceutical composition of the invention are formulated so as to allow the active ingredients contained therein to be bioavailable upon administration of the composition to a patient. Compositions that will be administered to a patient take the form of one or more dosage units, where for example, a tablet, capsule or cachet may be a single dosage unit, and a container of the compound in aerosol form may hold a plurality of dosage units.

Materials used in preparing the pharmaceutical compositions should be pharmaceutically pure and non-toxic in the amounts used. The inventive compositions may include one or more compounds (active ingredients) known for a particularly desirable effect. It will be evident to those of ordinary skill in the art that the optimal dosage of the active ingredient(s) in the pharmaceutical composition will depend on a variety of factors. Relevant factors include, without limitation, the type of subject (e.g., human), the particular form of the active ingredient, the manner of administration and the composition employed.

In general, the pharmaceutical composition includes a delivery vehicle of the present invention as described herein, in admixture with one or more carriers. The carrier(s) may be particulate, so that the compositions are, for example, in tablet or powder form. The carrier(s) may be liquid, with the compositions being, for example, an oral syrup or injectable liquid. In addition, the carrier(s) may be gaseous, so as to provide an aerosol composition useful in, e.g., inhalatory administration.

When intended for oral administration, the composition is preferably in either solid or liquid form, where semi-solid, semi-liquid, suspension and gel forms are included within the forms considered herein as either solid or liquid.

As a solid composition for oral administration, the composition may be formulated into a powder, granule, compressed tablet, pill, capsule, cachet, chewing gum, wafer, lozenges, or the like form. Such a solid composition will typically contain one or more inert diluents or edible carriers. In addition, one or more of the following adjuvants may be present: binders such as syrups, acacia, sorbitol, polyvinylpyrrolidone, carboxymethylcellulose, ethyl cellulose, microcrystalline cellulose, gum tragacanth or gelatin, and mixtures thereof; excipients such as starch, lactose or dextrins, disintegrating agents such as alginic acid, sodium alginate, Primogel, corn starch and the like; lubricants such as magnesium stearate or Sterotex; fillers such as lactose, mannitols, starch, calcium phosphate, sorbitol, methylcellulose, and mixtures thereof; lubricants such as magnesium stearate, high molecular weight polymers such as polyethylene glycol, high molecular weight fatty acids such as stearic acid, silica, wetting agents such as sodium lauryl sulfate, glidants such as colloidal silicon dioxide; sweetening agents such as sucrose or saccharin, a flavoring agent such as peppermint, methyl salicylate or orange flavoring, and a coloring agent.

When the composition is in the form of a capsule, e.g., a gelatin capsule, it may contain, in addition to materials of the above type, a liquid carrier such as polyethylene glycol or a fatty oil.

The composition may be in the form of a liquid, e.g., an elixir, syrup, solution, aqueous or oily emulsion or suspension, or even dry powders which may be reconstituted with water and/or other liquid media prior to use. The liquid may be for oral administration or for delivery by injection, as two examples. When intended for oral administration, preferred compositions contain, in addition to the present compounds, one or more of a sweetening agent, thickening agent, preservative (e.g., alkyl p-hydoxybenzoate), dye/colorant and flavor enhancer (flavorant). In a composition intended to be administered by injection, one or more of a surfactant, preservative (e.g., alkyl p-hydroxybenzoate), wetting agent, dispersing agent, suspending agent (e.g., sorbitol, glucose, or other sugar syrups), buffer, stabilizer and isotonic agent may be included. The emulsifying agent may be selected from lecithin or sorbitol monooleate.

The liquid pharmaceutical compositions of the invention, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or digylcerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

The pharmaceutical composition may be intended for topical administration, in which case the carrier may suitably comprise a solution, emulsion, ointment, cream or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device. Topical formulations may contain a concentration of the biologically active compound of from about 0.1 to about 25% w/v (weight per unit volume).

The composition may be intended for rectal administration, in the form, e.g., of a suppository which will melt in the rectum and release the drug. The composition for rectal administration may contain an oleaginous base as a suitable nonirritating excipient. Such bases include, without limitation, lanolin, cocoa butter and polyethylene glycol. Low-melting waxes are preferred for the preparation of a suppository, where mixtures of fatty acid glycerides and/or cocoa butter are suitable waxes. The waxes may be melted, and the aminocyclohexyl ether compound is dispersed homogeneously therein by stirring. The molten homogeneous mixture is then poured into convenient sized molds, allowed to cool and thereby solidify.

The composition may include various materials which modify the physical form of a solid or liquid dosage unit. For example, the composition may include materials that form a coating shell around the active ingredients. The materials which form the coating shell are typically inert, and may be selected from, for example, sugar, shellac, and other enteric coating agents. Alternatively, the active ingredients may be encased in a gelatin capsule or cachet.

The pharmaceutical composition of the present invention may consist of gaseous dosage units, e.g., it may be in the form of an aerosol. The term aerosol is used to denote a variety of systems ranging from those of colloidal nature to systems consisting of pressurized packages. Delivery may be by a liquefied or compressed gas or by a suitable pump system which dispenses the active ingredients. Aerosols of compounds of the invention may be delivered in single phase, bi-phasic, or tri-phasic systems in order to deliver the active ingredient(s). Delivery of the aerosol includes the necessary container, activators, valves, subcontainers, and the like, which together may form a kit.

The biologically active compounds may be in the form of the free base or in the form of a pharmaceutically acceptable salt such as the hydrochloride, sulfate, phosphate, citrate, fumarate, methanesulfonate, acetate, tartrate, maleate, lactate, mandelate, salicylate, succinate and other salts known in the art. The appropriate salt would be chosen to enhance bioavailability or stability of the compound for the appropriate mode of employment (e.g., oral or parenteral routes of administration).

A composition intended to be administered by injection can be prepared by combining the delivery vehicle of the present invention with water, and preferably buffering agents, so as to form a solution. The water is preferably sterile pyrogen-free water. A surfactant may be added to facilitate the formation of a homogeneous solution or suspension. Surfactants are compounds that non-covalently interact with the aminocyclohexyl ether compound so as to facilitate dissolution or homogeneous suspension of the aminocyclohexyl ether compound in the aqueous delivery system. Surfactants are desirably present in aqueous compositions of the invention because the aminocyclohexyl ether compounds according to the present invention may be hydrophobic. Other carriers for injection include, without limitation, sterile peroxide-free ethyl oleate, dehydrated alcohols, propylene glycol, as well as mixtures thereof.

Suitable pharmaceutical adjuvants for the injecting solutions include stabilizing agents, solubilizing agents, buffers, and viscosity regulators. Examples of these adjuvants include ethanol, ethylenediaminetetraacetic acid (EDTA), tartrate buffers, citrate buffers, and high molecular weight polyethylene oxide viscosity regulators. These pharmaceutical formulations may be injected intramuscularly, epidurally, intraperitoneally, or intravenously.

The present invention also provides kits that contain a pharmaceutical composition which includes one or more delivery vehicles. The kit also includes instructions for the use of the pharmaceutical. Preferably, a commercial package will contain one or more unit doses of the pharmaceutical composition. For example, such a unit dose may be an amount sufficient for the preparation of an intravenous injection. It will be evident to those of ordinary skill in the art that compounds which are light and/or air sensitive may require special packaging and/or formulation. For example, packaging may be used which is opaque to light, and/or sealed from contact with ambient air, and/or formulated with suitable coatings or excipients.

An "effective amount" of a CD inclusion complex delivery vehicle according to the invention includes a therapeutically effective amount or a prophylactically effective amount. A "therapeutically effective amount" refers to an amount effective, at dosages and for periods of time necessary, to achieve the desired therapeutic result. A therapeutically effective amount of a delivery vehicle may vary according to factors such as the disease state, age, sex, and weight of the individual, and the ability of the compound to elicit a desired response in the individual. Dosage regimens may be adjusted to provide the optimum therapeutic response. A therapeutically effective amount may also be one in which any toxic or detrimental effects of the delivery vehicle or active compound are outweighed by the therapeutically beneficial effects. A "prophylactically effective amount" refers to an amount effective, at dosages and for periods of time necessary, to achieve the desired prophylactic result. Typically, a prophylactic dose is used in subjects prior to or at an earlier stage of disease, so that a prophylactically effective amount may be less than a therapeutically effective amount. For any particular subject, the timing and dose of treatments may be adjusted over time (*e*.*g*., timing may be daily, every other day, weekly, monthly) according to the individual need and the professional judgment of the person administering or supervising the administration of the compositions.

In select embodiments, the present invention provides a composition or medicament that includes one or more biologically active molecules, selected from biologically active compounds or a solvate, pharmaceutically acceptable salt, ester, amide, complex, chelate, stereoisomer, stereoisomeric mixture, geometric isomer, crystalline or amorphous form, metabolite, metabolic precursor or prodrug thereof, including isolated enantiomeric, diastereomeric and geometric isomers thereof, and mixtures thereof, in combination with a pharmaceutically acceptable carrier, diluent or excipient, and further provides a method for the manufacture of such a composition or medicament.

Although various embodiments of the invention are disclosed herein, many adaptations and modifications may be made within the scope of the invention in accordance with the common general knowledge of those skilled in this art. Such modifications include the substitution of known equivalents for any aspect of the invention in order to achieve the same result in substantially the same way. Numeric ranges are inclusive of the numbers defining the range. The word "comprising" is used herein as an open-ended term, substantially equivalent to the phrase "including, but not limited to", and the word "comprises" has a corresponding meaning. As used herein, the singular forms "a", "an" and "the" include plural referents unless the context clearly dictates otherwise. Thus, for example, reference to "a thing" includes more than one such thing.

Citation of references herein is not an admission that such references are prior art to the present invention. Any priority document(s) and all publications, including but not limited to patents and patent applications, cited in this specification are incorporated herein by reference. All documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual publication were specifically and individually indicated to be incorporated by reference herein and as though fully set forth herein. The invention includes all embodiments and variations substantially as hereinbefore described and with reference to the examples and drawings.

In some embodiments, the invention excludes steps that involve medical or surgical treatment.

### EXAMPLES

### Example 1: Mucus Formula I

A cyclodextrin inclusion complex of camphene was prepared in 1:2 molar ratio of camphene to alpha cyclodextrin, utilizing a slurry method with food grade camphene from Vigon Corporation. Sustained release capsules of this camphene inclusion complex (6.5%) were made with the following formula per capsule:

| | |
|---|---|
| Camphene inclusion | 400 mg (26 mg net) |
| K-100M cellulose (Colorcon) | 80 mg |
| E4M cellulose (Colorcon) | 20 mg |
| Amylase enzyme | 4 mg |
| Calcium Laurate (flow enhancer) | 4 mg |

### Example 2: Mucus Formula II

Additional cyclodextrin inclusion complex formulations were prepared for addition to Mucus Formula I, as follows:
Peppermint oil (NOW Foods) in 1:4 molar ratio of oil to gamma cyclodextrin, utilizing the slurry method.
Eucalyptol (Vigon) in 1:1 molar ratio of eucalyptol to alpha cyclodextrin, utilizing the slurry method.

The foregoing peppermint oil and eucalyptol inclusion complex formulations were added to the components of Mucus Formula I, to provide capsules of Mucus Formula II:

| | |
|---|---|
| Camphene inclusion (6.5%) | 150 mg (10 mg net) |
| Eucalyptol inclusion (10%) | 135 mg (13.5 mg net) |
| Peppermint oil inclusion (15%) | 46 mg (7 mg net) |
| K-100M cellulose (Colorcon) | 70 mg |
| E4M cellulose (Colorcon) | 17 mg |
| Amylase enzyme | 4 mg |

### Example 3: Mucositis Associated with Nonalcoholic Steatohepatitis (NASH)

Subject BC had a history of liver cancer, and resulting mucositis associated with nonalcoholic steatohepatitis (NASH, a subset of the progressive form of nonalcoholic fatty liver diseases), in conjunction with cirrhosis. BC underwent liver surgery for intrahepatic cholangiocarcinoma, followed by cancer treatments that included chemotherapy and radiation. A side effect of the radiation was the development of mucositis (excess production of mucus), which was recalcitrant to conventional therapies.

BC commenced treatment with the Mucus Formula II of Example 2 on day 1, at a dosage of 3 capsules 3 times daily. By day 5, respiratory mucus was dramatically ameliorated, with marked change in lung congestion although accompanied by persistent cough. By day 10 of treatment with the capsules, excess respiratory mucus had virtually disappeared symptomatically, and coughing was largely resolved. Continued treatment with the capsules provided lasting amelioration of the symptoms of mucositis, providing evidence of surprising efficacy in treatment of airway mucus dysfunction, including antitussive efficacy.

### Example 4: Bronchiectasis

50 year old subject D had a longstanding diagnosis of severe and intractable bronchiectasis, and had been pseudomonas positive on lung sputum for ~20 yrs. The patient was undergoing treatment with Augmentin (amoxicillin and clavulanate) for sinus infection and nebulized hypersol, albuterol and Colistin antibiotic (28 days-on, then 28 days-off).

The patient began treatment with Mucus Formula II on June 11th at 4 capsules two times a day. On June 14, dosing was increased to 6 capsules two times a day, and this was accompanied by a transition to sleeping through the night, attributed to a resolution of longstanding symptoms of coughing associated with being in a prostrate or prone position. By June 17th, D was sleeping well with continued improvement in airway mucus, an outcome attributed by the patient to the resolution of lung congestion mediated by the Mucus Formula II treatment. By June 19th, D reported continued improvement, sleeping through the night consistently. On June 20th, D reported expectorant effects, with airway mucus thinner, easier to cough out all day, with mucus lighter green to clear, even during the worst part of the "off 28 days" portion of the cycle of treatment with the nebulized antibiotic (the last two weeks of that cycle during which D had typically experience airway mucus turning a dark green). D reported reduced chest pressure, productive cough, enabling horizontal sleep through the night. D had been sleeping through the night since the first day of taking Mucus Formula II, June 12th. This was a surprising result in the face of what had been a consistent, chronic history of sleeping no more than 2-3 hours at a time before being awakened by coughing, regardless of sleeping position or incline. This was a dramatic and consistent change after years of intermittent sleep in a recliner.

On June 30th, D continued treatment switching to Mucus Formula II with added guaiol and fenugreek oil, designated Mucus Formula II⁺. By July 3rd, D reported continued significant improvement in airway mucus dysfunction, with less cough, more productive cough, and less mucus. Improvements included decreases in frequency, duration and intensity of cough, with cough more productive but with less mucus overall to manage. At this point, patient D was two weeks into the "on" part of the 28 days-on, 28 days-off cycle of antibiotics. Mucus is white, with an occasional tinge of yellow. Typically at this point in the antibiotic cycle mucus is yellow to lighter yellow (never clear/white). D continues to sleep through the night. By July 10th, at the beginning of the fourth week of the "on" portion of the antibiotic cycling, there was continued improvement and D reported being able to sleep supine for the first time in many years, with white mucus (no yellow) and less mucus. Noticable expectorant effects, with mucus much easier to move even than previous week.

This example illustrates effective treatment of airway mucus dysfunction with oral camphene cyclodextrin formulations, including formulations that further include guaiol and fenugreek oil.

### Example 5: Viral Pneumonitis, Mucoactive Effects

A composition comprising the following combination of CD inclusions was provided in capsule form for oral administration:
Extract of Eucalyptus *(Myrtaceae)* standardized for eucalyptol 6 mg (in beta CD inclusion complex);
Extract of *Syncarpia glomulifera* standardized for camphene 6 mg (in beta CD inclusion complex);
Peppermint *(Mentha piperita)* oil 6 mg (in beta CD inclusion complex);
Extract of *Pinus* (*Pinaster and*/*or Elliotii* and/or *Sylvestris*] standardized for carene delta 3 4mg (beta CD or gamma CD inclusion);
Fenugree (Trigonella foenum-graecum) extract (Fenugreek Absolute) for 4 mg (in gamma CD inclusion complex);
Extract of *Bulnesia sarmientoi* standardized for guaiol for 4 mg (in gamma CD inclusion complex);
K250 time release agent 80 mg;
Amylase 7 mg; and,
Ca Laurate 5 mg.

An adult male patient was diagnosed with a viral infection, exhibiting dry, unproductive cough to the point of vomiting. The patient initiated treatment with 4 capsules of the foregoing formulation, 2 times a day for 2 days. The patient's cough became productive, with evident mucoactive effects from the formulation leading to an improvement in the patient's sense of well being.

### Example 6: Erectogenic Effects

Male subject A began dosing Mucus Formula I, 3 capsules twice daily. Within 48 hours, breathing with exercise had markedly improved with a noticeable decline in respiratory mucus. Within 48 hours of initial dosing with Mucus Formula I, subject noted the occurrence of nocturnal penile tumescence, which the 67 year old subject had not been experiencing for some time. Subject increased Mucus Formula I dosage to 4 capsules twice daily, with the immediate effect that nocturnal penile tumescence, as well as susceptibility to diurnal or nocturnal erectogenic stimulation, was significantly enhanced. Subject gauged the erectogenic effect to be equivalent to 70-85% of the effect of a 25-50 mg dosage of sildenafil. Subject further found that when on a Mucus Formula I dosage of 4 capsules twice daily, a dosage of 5 milligrams of sildenafil achieved virtually the same erectogenic potentiation as past dosing with 25 - 50 milligrams sildenafil, with none of the side effects previously experienced with 25 - 50 milligrams of sildenafil (which may for example include headache, flushing, upset stomach, abnormal vision, stuffy or runny nose, back pain, muscle pain and nausea).

## Claims

1. A cyclodextrin (CD) inclusion complex formulation, comprising:
extract of eucalyptus (Myrtaceae) comprising eucalyptol in a CD inclusion;
extract of Syncarpia glomulifera comprising camphene in CD inclusion;
peppermint (Mentha piperita) oil in a CD inclusion;
extract of Pinus comprising carene delta 3 in a CD inclusion;
fenugreek extract in a CD inclusion; and,
extract of Bulnesia sarmientoi comprising guaiol in a CD inclusion.

2. The formulation of claim 1, further comprising:
a time release agent; optionally a K250 time release agent.

3. The formulation of claim 1 or 2, further comprising a cyclodextrin degrading enzyme.

4. The formulation of claim 3, wherein the enzyme is an amylase, a cyclodextrinase, a maltogenic amylase or a neopullulanase.

5. The formulation of claim 3, wherein the enzyme is a mammalian salivary amylase, a mammalian pancreatic amylase or a microbial amylase.

6. The formulation of any one of claims 1 to 5, wherein the cyclodextrin (CD) in each inclusion is an alpha cyclodextrin, beta cyclodextrin or gamma cyclodextrin.

7. The formulation of any one of claims 1 to 6, further comprising calcium laurate.

8. The formulation of any one of claims 1 to 7, wherein:
the extract of eucalyptus (Myrtaceae) comprises approximately 4-8 mg, optionally 6 mg, eucalyptol; and/or,
the extract of Syncarpia glomulifera comprises approximately 4-8 mg, optionally 6 mg, camphene; and/or,
the peppermint (Mentha piperita) oil is provided in approximately 4-8 mg, optionally 6 mg, in the CD inclusion; and/or,
the extract of Pinus comprises approximately 2-6 mg, optionally 4 mg, carene Delta 3; and/or,
the fenugreek extract is provided in approximately 2-6 mg, optionally 4 mg, in the CD inclusion; and/or,
the extract of Bulnesia sarmientoi comprises approximately 2-6 mg, optionally 4 mg, guaiol.

9. The formulation of any one of claims 1 to 7, wherein:
the extract of eucalyptus *(Myrtaceae)* CD inclusion comprises a eucalyptol beta CD inclusion; and/or,
the extract of *Syncarpia glomulifera* CD inclusion comprises a camphene beta CD inclusion; and/or,
the peppermint *(Mentha piperita)* oil is provided at least partly in a beta CD inclusion; and/or,
the extract of *Pinus* CD inclusion comprises a carene Delta 3 beta CD inclusion; and/or,
the fenugreek extract is provided at least partly in a gamma CD inclusion; and/or,
the extract of *Bulnesia sarmientoi* CD inclusion comprises a guaiol gamma CD inclusion.

10. The formulation of any one of claims 1 to 7, further comprising humulene.

11. The formulation of any one of claims 1 to 10, for use as a mucoactive agent to treat an airway disorder in a subject in need thereof.

12. The formulation for use of claim 11, wherein the airway disorder is a respiratory disease or condition **characterized by** oversecretion or inspissation of mucus.

13. The formulation for use of claims 11 or 12, wherein the airway disorder is asthma, chronic obstructive pulmonary disease (COPD), cystic fibrosis (CF), CF related bronchiectasis, non-CF bronchiectasis, viral bronchiolitis, bacterial bronchiolitis, or a microbial (optionally bacterial or viral) infection.
